# EUROPEAN PATENT APPLICATION

(11) **EP 4 838 205 A1**
(43) Date of publication of application: **11.11.2026**
(21) Application number: 25191406.5
(22) Date of filing: 23.07.2025
(51) Int. Cl.: G01N 21/25, G01N 21/31, G01N 21/3504, G01N 21/359, G01N 21/39, G01N 33/00

(54) **SYSTEM FOR PASSIVE OPTICAL MONITORING OF GAS CONCENTRATION AND PHYSICAL PARAMETER, AND OPERATION METHODS THEREOF**

(30) Priority: 08.05.2025 PT 2025120251
(71) Applicant: INESC TEC - Instituto de Engenharia de Sistemas e Computadores, Tecnologia e Ciência, 4200-465 Porto (PT)
(72) Inventor: FLORIDIA, CLAUDIO, 4200-465 PORTO (PT); COSTA COELHO, LUIS CARLOS, 4200-465 PORTO (PT); SANTINI, LORENZO, 4200-465 PORTO (PT)
(74) Representative: Patentree

(57) **Abstract**

The present disclosure relates to a system for passive optical monitoring of a gaseous substance at a plurality of measuring points, comprising a laser interrogator for emitting a laser pulse and receiving laser pulses; a plurality of fibre-optic lines, each fibre-optic line for a measuring point; an optical splitter connected to the laser interrogator for splitting the emitted laser pulse into the plurality of fibre-optic lines; a plurality of gas concentration sensors, each connected to a corresponding fibre-optic line; and a data processor configured for determining a presence at a measuring point of the gaseous substance to be monitored from an attenuation of a corresponding received laser pulse; wherein each fibre-optic line has a unique delay in respect to the other fibre-optic lines; wherein each gas concentration sensor comprises a channel; an end of a corresponding fibre-optic line; and a mirror.

## Description

### TECHNICAL FIELD

The present disclosure relates to a system for monitoring a concentration of a gaseous substance and, optionally, another physical parameter, in particular with application for the mining sector, oil and gas extraction sector, landfill monitoring, or any industrial field where multipoint gas monitoring may be required.

### BACKGROUND

Industries that extract or make use of gases, e.g. methane, for their industrial processes are in a pivotal situation. For the extraction industry, a greater complexity of exploring new fields is observed, while other industries that make use of the extracted gases have an increasing multitude of final products where said gases are required. These complexities are associated with a data drive to digitalize all industrial sectors, aiming to increase overall competitiveness, improving the safety of operations and mitigating possible environmental damage.

Furthermore, global climate change is correlated with the emission of greenhouse gases, such as those from the production and use of energy sources and the transformation of energy, namely fossil fuels, coal, oil and natural gas, which represent a significant share in global greenhouse gas emissions. Fugitive emissions are leaks in equipment arising from mechanical operating failures, wear of sealing structures and inherent characteristics of the materials. For instance, whenever dealing with natural gas transport systems, these emissions are mainly composed of methane, which reduce the energy efficiency of processes and intensify climate change.

For these reasons, there has been a focus on the creation and implementation of gas monitoring systems for industrial complexes.

Document WO2024145702A1 discloses a system and method for passive optical monitoring of the concentration of multiple gases and/or multiple parameters at various points for obtaining and monitoring the methane concentration in offshore platforms and onshore structures, and also in the fields of mining, landfills and other industrial sectors. The system consists in the sending, by a fibre optic unit, of a frequencymodulated light pulse (intra-pulse modulation) that is optically selected and directed at optical splitters and delay lines for measurements at various points, giving rise to multiple signals that interact with the methane gas in reflective sensors, that is, sensors that reflect light. This reflected light returns to the fibre optic unit and is detected by a single optical receiver and the time signals of the aforementioned light are analysed individually by calculating the fast Fourier transform (FFT). The FFT signal thus obtained contains an amplitude A0 at the zero frequency or DC component, f0, an amplitude A1 at the same modulation frequency as the laser, f1, and an amplitude A2 at twice the original laser frequency, f2 = 2 x f1. In this way, the system makes it possible to obtain the concentrations (C) from the N sensors on the basis of the amplitudes A0, A1 and A2.

Systems, such as the one previously mentioned, require a high laser modulation rate with concomitant tuning in the center of the line, which is more difficult and costly to achieve. Furthermore, there is a need for acquisition with a large sampling rate, consequently limiting the pulse width and, therefore, the need for longer delay optical fibers that, when employed in many points, increase the total number of kilometers of employed fiber in the overall system, yielding higher costs. Long optical fibers, e.g. 250 m, also take up physical space, and are difficult to make or acquire in the exact length on the market. This may cause the overlap of return pulses from the sensors to the fiber optic unit, while also requiring A large space in the passive boxes, with optical splitters and the delay lines.

Systems, such as the one previously mentioned, require complex acquisition and control electronics. For instance, there is a need for a Wavelength Division Multiplexing (WDM) coupler for each sensor, to enable the separation between the Fiber Bragg Grating (FBG) signal, occurring at wavelengths similar to those of the C-band, and the signals for detecting a gas concentration, e.g., 1650.9 nm for a methane gas.

These facts are disclosed in order to illustrate the technical problem addressed by the present disclosure.

### GENERAL DESCRIPTION

The present disclosure relates to a system and method for passive optical monitoring of the concentration of a gas in a gas flow, such as methane, as well as the monitoring of multiple parameters at multiple points in offshore platforms and onshore structures, or in the fields of mining, landfills and other industrial sectors.

In an embodiment, the method for gas concentration measurement consists in sending, by a remote fibre optic unit, light pulse that is optically selected and directed at optical splitters and delay lines for various measurement points of interest, giving rise to multiple signals that interact with the methane gas in reflective sensors, that is, sensors that reflect light. These reflected light pulses return to the remote unit a time signal from N point sensors, which are detected by a single optical receiver and their respective time signals are analysed individually to obtain its reflected amplitude. Each pulse of light is first tuned to one the monitored gas absorption line, for example 1650.9 nm for methane, to obtain the absorption that occurs at each sensor cells. Afterwards, the laser is tuned out of this absorption band, where no methane absorption occurs to monitor relative amplitude at each N point sensors. By making the difference between these two signals, for each time slice relative to each sensor, it is possible to obtain the correct amount of absorption and thus the concentration of methane.

In an embodiment, the measurements done outside of the monitored gas absorption band are periodically made after each measurement done at the wavelength corresponding to the absorption line, or after 10 measurements.

In an embodiment, another laser may be present which is always tuned outside the absorption band of the gas, and is periodically triggered to accomplish the measurements.

In an embodiment, the system enables concomitant measurement by using a Fiber Bragg Grating (FBG) interrogator, which emits signals in another optical band, generally using the C band (1530 nm to 1565 nm), but may be any band not coincident with absorption of the gas. The disclosed can be used for the monitoring of a range of gases depending on their absorption lines and the choice of the FBGs sensors and band.

In an embodiment, the proposed system is modular and scalable, and the configuration is reflexive.

In an embodiment, the gas sensor is a collimator (i.e., a lens) to expand the light from the optical fiber to a mirror. The mirror within the gas sensor has its reflection just around the wavelength of the gas absorption. The mirror reflects the tuned and untuned wavelengths, that is, for example, 1650.9 nm ± 1nm (or even ± 50 nm) if the gas flow boing monitored is a methane flow. The reflected wavelengths does not coincide with the wavelengths of the FBGs.

In an embodiment, there is compensation of optical loss with the fiber links in the light pulse measurements, and this is done by measuring both in the absorption line and outside said absorption line, for each sensor of the network.

In an embodiment, the system enables measuring gas concentration at multiple points, supporting large-scale expansion in a modular and scalable way.

In an embodiment, the system, avoids the use of reception systems with multiple detectors, thereby reducing complexity and cost. The system operates in a reflection configuration, which enables the use of a single detection at the same "side" of the interrogation unit, simplifying the overall system.

In an embodiment, the system incorporates mechanisms to compensate for factors that degrade the quality of the optical signal.

In an embodiment, simultaneous measurement of multiple physical quantities occur by allowing the concurrent measurement of gas concentration and other physical quantities using the same optical network.

It is disclosed a system for passive optical monitoring of a gaseous substance at a plurality of measuring points, comprising:
a laser interrogator for emitting a laser pulse and receiving laser pulses;
a plurality of fibre-optic lines, each fibre-optic line for a measuring point;
an optical splitter connected to the laser interrogator for splitting the emitted laser pulse into the plurality of fibre-optic lines;
a plurality of gas concentration sensors, each connected to a corresponding fibre-optic line; and
a data processor configured for determining a presence at a measuring point of the gaseous substance to be monitored from an attenuation of a corresponding received laser pulse;
wherein each fibre-optic line has a unique delay in respect to the other fibre-optic lines, i.e., each fibre-optic line has a delay that is different from the delay of any of the other fibre-optic lines;
wherein each gas concentration sensor comprises:
   a channel for receiving the gaseous substance to be monitored by absorption by the gaseous substance of a corresponding split laser pulse;
   an end of a corresponding fibre-optic line for sending the split laser pulse through the channel; and
   a mirror for reflecting the emitted laser pulse back into the corresponding fibre-optic line.

In an embodiment, the system further comprises:
a Fiber Bragg Grating (FBG) interrogator, for emitting and receiving an FBG signal;
a plurality of FBG sensors arranged for measuring a physical parameter by receiving the emitted FBG signal and reflecting the emitted FBG signal, according to the physical parameter to be measured, towards the FBG interrogator; and
a Wavelength Division Multiplexing, WDM, coupler arranged for multiplexing the emitted FBG signal and the emitted laser pulse into the optical splitter, and for demultiplexing the reflected FBG signals and the reflected laser pulses from the optical splitter.

In an embodiment, the emitted FBG signal and the emitted laser pulse have different wavelengths.

In an embodiment, the mirror of the gas concentration sensor is configured to not reflect the emitted FBG signal.

In an embodiment, the physical parameter measured by the plurality of FBG sensors is selected from: temperature, strain, pressure, water level, current, a second gas substance concentration, or combinations thereof, in particular, the second gas substance being Hydrogen.

In an embodiment, the laser interrogator is configured to emit and receive a calibration laser pulse having a wavelength that does not correspond to an absorption band of the gaseous substance, and the data processor is further configured for calibrating the presence at the measuring point of the gaseous substance to be monitored from an attenuation of the received calibration laser pulse.

In an embodiment, one of said fibre-optic lines has no added delay line and each of the remaining fibre-optic lines comprises a delay line wherein each delay line has a unique delay in respect to the other delay lines, i.e., each delay line has a delay that is different from the delay of any of the other delay lines.

In an embodiment, the laser interrogator comprises:
one or two Distributed Feedback Lasers (DFL) for generating a laser signal;
one or two laser drivers for controlling each of the one or DFL by sending an electrical control signal;
a gaseous-substance reference absorption cell, comprising the gaseous substance to be monitored by absorption of the gaseous substance of a corresponding generated laser signal;
a first photodetector arranged for detecting the generated laser signal from the gaseous-substance reference absorption cell, and for converting said portion of laser signal into an electrical signal;
a second photodetector arranged for detecting at least one reflected laser pulse and converting said received laser pulse into at least one electrical pulse signal;
a signal acquisition processor configured to acquire the electric signal from the first photodetector, and to acquire the at least one electrical signal pulse from the second photodetector;
the data processor configured to quantify the concentration of the monitored gas from the acquired electric signal from the second photodetector, and to calibrate the one or two laser drivers from the acquired electric signal from the first photodetector;
a pulse generator for generating a further electrical signal pulse of a predefined pulse width, and for triggering the signal acquisition processor;
a fast switch for generating the laser pulse from the laser signal generated by the one or two DFL, wherein the laser pulse has the same pulse width as the further electrical signal;
a first optical splitter for splitting the generated laser signal from the one or two DFL into two identical outputting laser signals, wherein one of the two identical outputting laser signals is sent to the fast switch, and wherein another of the two identical outputting laser signals is sent to the gaseous-substance reference absorption cell; and
an optical circulator arranged for receiving the generated laser pulse from the fast switch and transmitting said generated laser pulse to the WDM coupler, and for receiving the demultiplexed laser pulses and transmitting said demultiplexed laser pulses to the second photodetector.

An embodiment comprises a module further comprising one or more optical splitters for connecting the WDM coupler to the two or more gas concentration sensor and the two or more FBG sensors, wherein the splitter has a number of outputs identical to the number of the two or more gas concentration sensor and the two or more FBG sensors.

In an embodiment, the module further comprises at least one delay line connected between the one or more optical splitters and each of the two or more FBG sensors, for delaying the receiving laser pulses and the receiving light pulses.

In an embodiment, each gas concentration sensor of plurality of gas concentration sensors further comprises an optic, arranged on an end of the corresponding fibre-optic line, for sending the split laser pulse through the channel to be reflected by the mirror back into the corresponding fibre-optic line.

In an embodiment, the mirror of the at least one gas concentration sensor reflects rays of the at least two distinct wavelengths, emitted by the one or two DFL.

In an embodiment, the first optical splitter for of the gas concentration interrogator divides the laser signal, generated by one of the one or two DFL, into two outputting laser signals, wherein:
one of the two outputting laser signals, comprises a smaller power percentage of the laser signal generated by the one of the one or two DFL, which is sent to the gas reference absorption cell, and
another of the two outputting laser signals, comprises a larger power percentage of the laser signal generated by the one of the one or two DFL, which is sent to the fast switch.

An embodiment further comprises two or more optical splitters; and further delay lines arranged between the WDM coupler and the module, for delaying the receiving laser pulses and the receiving light pulses.

In an embodiment, the fast switch can be selected from one of the following elements: an optical switch; an optical modulator; a Semiconductor Optical Amplifier, SOA; a Booster Optical Amplifier, BOA; or similar optical device.

In an embodiment, the gas concentration interrogator comprises two DFL and two laser drivers, wherein:
the laser signal generated by the one of the two DFL has an identical wavelength of an absorption peak of the gas; and
the laser signal of the another of the two DFL has a different wavelength of the absorption peak of the gas; and
each of the two laser drivers controls each of the two DFL.

In an embodiment, the gas concentration interrogator further comprises two fast switches and a second optical splitter, wherein:
one of the two fast switches is connected to the first optical splitter of the gas concentration interrogator, for generating a first laser pulse from the laser signal generated by the one of the two DFL;
another of the two fast switches is connected to another of the two DFL of the gas concentration interrogator, for generating a second laser pulse from the laser signal generated by the another of the two DFL; and
a second optical splitter connecting each of the two fast switches to the optical circulator.

In an embodiment, the fast switch of the gas concentration interrogator receives the laser signal, with a wavelength identical to that of the absorption peak of the gas, from the first optical splitter, generated by the one of the two DFL, and receives the laser signal, with a different wavelength to that of the absorption peak of the gas, from the other of the two DFL.

It is also disclosed a method for passive optical monitoring of a gaseous substance at a plurality of measuring points, comprising the following steps:
generating a laser pulse in the gas concentration interrogator with an identical wavelength of an absorption peak of the gas;
converting the at least one demultiplexed laser pulse with an identical wavelength of an absorption peak of the gas, from the at least one gas concentration sensor into at least one electrical pulse signal with the second photodetector;
sending the at least one electrical pulse signal to the signal acquisition processor;
generating a laser pulse in the gas concentration interrogator different wavelength of the absorption peak of the gas;
converting the at least one receiving laser pulse with another wavelength, from the at least one gas concentration sensor into at least one electrical signal with the second photodetector.

In an embodiment, the generation of a laser pulse for the measurement of a gas concentration comprises the following steps:
sending an electrical control signal from the one or two laser drivers to the one or two DFL for generating a laser signal;
sending the generated laser signal from the one or two DFL to the first optical splitter for dividing the generated laser signal into two outputting laser signals,
wherein:
   one of the two outputting laser signals comprises a smaller power percentage relative the laser signal generated, and
   another of the two outputting laser signals comprises a larger power percentage relative the laser signal, which is sent to the fast switch; and
   generating a laser pulse from the laser signal generated by the one or two DFL, wherein the laser pulse has the same pulse width as the further electrical signal pulse generated from the pulse generator.

### BRIEF DESCRIPTION OF THE DRAWINGS

The following figures provide preferred embodiments for illustrating the disclosure and should not be seen as limiting the scope of invention.
**Figure 1****:** Schematic representation of the general architecture of an embodiment of the disclosure.
**Figure 2****:** Schematic representation of a preferred embodiment of the disclosure.
**Figure 3A****:** Schematic representation of a gas absorption plot.
**Figure 3B****:** Schematic representation of a time diagram with the emitted and received pulses.
**Figure 4****:** Schematic representation of an alternative embodiment of the gas concentration interrogator with two laser drivers and a 2x1 optical switch.
**Figure 5****:** Schematic representation of an alternative embodiment of the gas concentration interrogator with two laser drivers, two optical switches and one optical splitter 50:50.
**Figure 6****:** Schematic representation of an embodiment of the gas concentration sensor.
**Figure 7****:** Schematic representation of a modular and scalable embodiment of the disclosed.
**Figure 8****:** Table with possible combinations of number and kinds of splitters, as well as the respective total amount of delay line.
**Figure 9****:** Schematic representation of an embodiment with optical switches and with subnetworks of sensors and respective accompanying devices.

### DETAILED DESCRIPTION

The present disclosure relates to a system for measuring a specific gas flow and any second physical quantity, for the mining sector, oil and gas extraction sector, landfill, or any industrial field where multipoint gas monitoring is required.

The present disclosure relates to a system for passive optical monitoring of a gaseous substance at a plurality of measuring points, comprising a laser interrogator for emitting a laser pulse and receiving laser pulses; a plurality of fibre-optic lines, each fibre-optic line for a measuring point; an optical splitter connected to the laser interrogator for splitting the emitted laser pulse into the plurality of fibre-optic lines; a plurality of gas concentration sensors, each connected to a corresponding fibre-optic line; and a data processor configured for determining a presence at a measuring point of the gaseous substance to be monitored from an attenuation of a corresponding received laser pulse; wherein each fibre-optic line has a unique delay in respect to the other fibre-optic lines; wherein each gas concentration sensor comprises a channel; an end of a corresponding fibre-optic line; and a mirror.

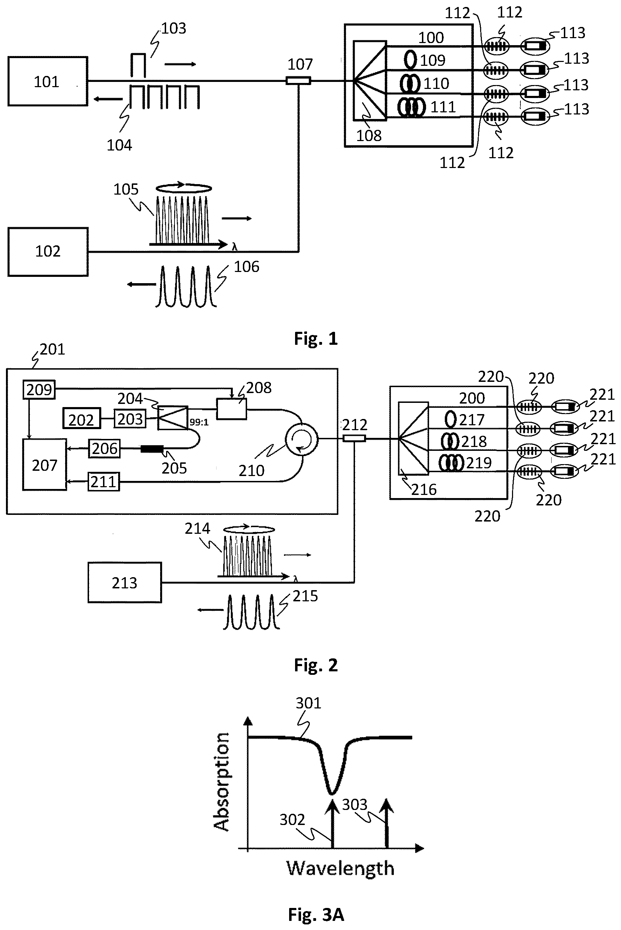

**Figure 1** represents a schematic representation of the general architecture of an embodiment of the disclosure, where: **101** represents a gas concentration interrogator; **102** represents a Fiber Bragg Grating (FBG) interrogator; **103** represents the generated optical pulse by the gas concentration interrogator; **104** represents the reflected optical pulses; **105** represents the scanning light wavelengths emitted from the FBG interrogator; **106** represents the received light wavelengths; **107** represents Wavelength Division Multiplexing (WDM) coupler; **108** represents an Optical Splitter (OS); **109, 110** and **111** represent one, two and three Delay Lines (DL), respectively; **112** represents a FBG sensor; and **113** represents a gas concentration sensor.

In an embodiment, a gas concentration interrogator, **101,** sends a single optical pulse, **103,** with a defined Pulse Width (PW) expressed in units of time, for example PW = 100 ns. This optical pulse travels through an optical fiber cable to a Wavelength Division Multiplexing (WDM) coupler, **107,** that unites the signal from the gas concentration signal, generated with a specific wavelength and the signal, **105,** from a Fiber Bragg Grating (FBG) interrogator, **102,** usually in the C-band (1530 nm to 1565 nm). This optical pulse goes through an Optical Splitter (OS), **108,** that can be of various ratios. In the exemplary embodiment represented in Figure 1, the ratio of the OS is of 1:4, but it can be of 1:32, 1:64 or even 1:128. The signal with a wavelength of 1650 nm is then split and each division travels a different distance, due to the presence of Delay Lines (DL), **109, 110** and **111,** of predetermined size. For a pulse of PW of 100 ns the DL is equal or greater than 10 meters, to avoid reflections from gas concentration sensors, **112,** overlapping to each other. These reflected pulses, **104,** return to the gas concentration interrogator, **101,** carrying information of the absorption due to the monitored gas in the gas concentration sensors. This will be explained afterwards.

A preferred embodiment can measure other parameters of interest such as temperature, strain, pressure, water level, current, etc. by using well-known fiber Bragg grating technologies. In this case an FBG interrogator, **102,** of any kind (here illustrated as scanning wavelength technology based) is used. The signal from this FBG interrogator, **102,** travels on its own fiber optical cable and is united with the gas concentration interrogation unit signal in the WDM, **107;** signal travels together to the OS, **108,** and is divided in the many branches (in the case of **Figure 1****,** the signal is divided in four branches). Each branch has a FBG sensor, **112,** that reflects a specific wavelength. The rest of the signal is lost in the mirror of the gas sensor, for instance, **116,** that reflects only in the band for the specific wavelength. For instance, in the case of methane gas monitoring, the ideal wavelength is of 1650 nm. These FBG signals, **106,** return to the FBG interrogator, **102,** and have their wavelength displayed or read by the device. Even if **Figure 1** shows one FBG per branch, a series of FBGs can also be used in each branch, as known by those skilled in the art.

In an embodiment, the gas concentration interrogator, **101,** FBG interrogator, **102,** and WDM coupler, **107,** can be in a single place, while the splitter, **108,** with delay lines, **109, 110** and **111,** is placed remotely. Furthermore, optical switches can be used locally or remotely, as explained further on.

**Figure 2** represents a schematic representation of a preferred embodiment of the disclosure, where: **201** represents a gas concentration interrogator; **202** represents a laser driver; **203** represents a Distributed Feedback Laser (DFL), **204** represents a 1:99 Splitter; **205** represents gas reference absorption cell (in the previous example, this would be a methane reference absorption cell); **206** represents a first photodetector; **207** represents a data acquisition card; **208** represents an fast switch; **209** represents a pulse generator; **210** represents an optical circulator; **211** represents a second photodetector; **212** represents a WDM coupler; **213** represents a FBG interrogator; **214** represents the emitted light wavelengths; **215** represents the received light wavelengths; **216** represents an Optical Splitter (OS); **217, 218** and **219** represent three DL of different lengths, respectively; **220** represents a FBG sensor; and **221** represents a gas concentration sensor.

The preferred embodiment shown in **Figure 2****,** describes the gas concentration interrogator, **201.** A laser driver, **202,** controls a DFB, **203.** The control is usual by those skilled in the art. Control of temperature by a Thermo-Electric Cooler (TEC) with feedback by a thermistor, also controls the laser current, which is used to set the laser in the absorption line of gas being monitored. There are many lasers, as those skilled in the art know well. Here, the laser wavelength of 1650.9 nm is used for the methane, due to the high availability of telecommunication lasers at this wavelength. To guarantee that laser is tuned in the exact peak of the absorption the signal, part of the signal is split, for example by a 1:99 splitter, **204,** where the 1% of the signal passes through a gas reference absorption cell, **205,** for instance a CH₄ absorption cell, and detected by a first photodetector, **206,** and acquired by a Data Acquisition Card (DAQ), **207.** This DAQ, **207,** may be a fast acquisition card or oscilloscope, triggered by a pulse generator, **209.** The signal of the first photodetector, **206,** is used as feedback to fine tune the DFB, **203,** in the wavelength that gives the maximum absorption, minimizing the signal outputted by the first photodetector, **206.**

In an embodiment, a Fast Switch (FS), **208,** which can be an optical switch, an optical modulator, a Semiconductor Optical Amplifier (SOA) or a Booster Optical Amplifier (BOA) or similar optical device, is used to pulse the laser with the previously mentioned pulse width, driven by the pulse generator, **209.** The signal is sent to an optical circulator, **210,** and the procedure described previously in regards to **Figure 1** takes place. The pulse replicas, which are as many as the division present in the OS, **216,** return to the optical circulator, **210,** and are redirected to a second photodetector, **211,** that yield a respective electrical signal to the DAQ, **207,** which is triggered by the pulse generator, **209.** To obtain enough data within each pulse of a given PW, the DAQ, **207,** acquires a minimum of 1 sample per pulse, but preferably at least 10 samples per pulse. This means that, for PW = 100 ns and a number of samples per pulse (NS) of 10 (NS = 10), a sample rate (SR) of 10 samples/100 ns = 100 000 000/s = 100 MS/s (100 megasamples per second) is required. Thus, there is a relationship between variables PW, DL and SR, such that $DL \left(meters\right) = PW \left(ns\right) / 10$ and; $SR \left(S/s\right) = NS / PW \left(s\right)$ or $SR \left(MS/s\right) = 1000 \times NS / PW \left(ns\right) .$

In an embodiment, the first equation, relating the Pulse Width, PW, with the length of a Delay Line, DL, for instance, **217,** originates from the estimated light velocity in a fiber optic line. Considering that the refractive index, n, of a fiber line is of n ≈ 1.5, and that n = c/v, where c is the velocity of light in a vacuum (c ≈ 3.0 × 10⁸ m/s). The velocity of light in a fiber optic, v, is then of approximately 2.0 × 10⁸ m/s. In this manner, and considering that any light and/or laser pulse travels through the delay line, **217,** in both directions, one can estimate that 10 meters of delay line permits the travelling of a light and/or laser pulse of 100 ns.

**Figure 3A** schematically represents a gas absorption plot, where: **301** is the absorption curve of the monitored gas with a peak of no absorption; **302** is the radiation wavelength nor absorbed by the gas being monitored; **303** is the radiation wavelength that is absorbed by the gas being monitored.

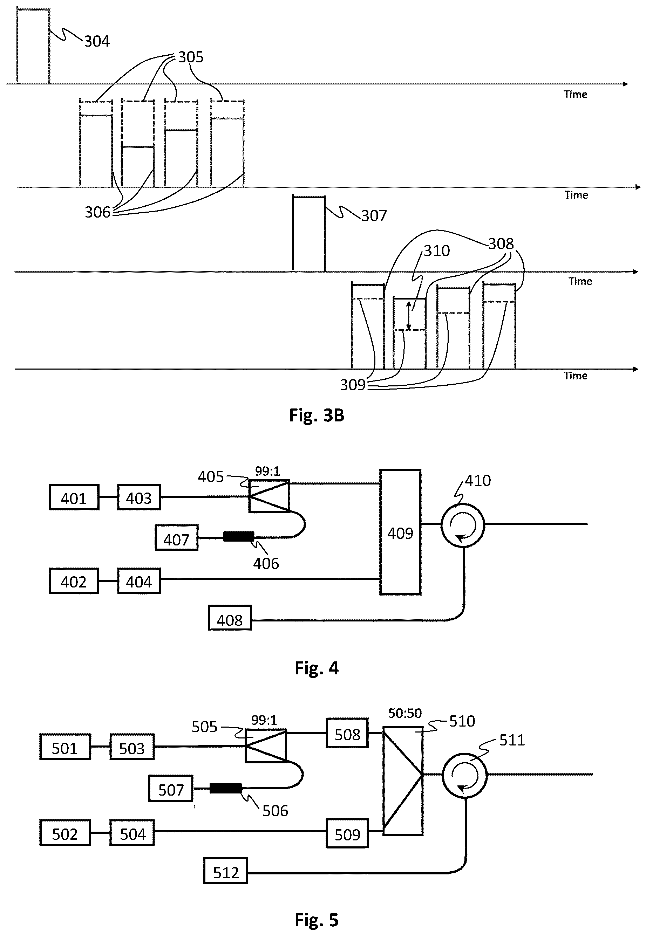

**Figure 3B** schematically represents a time diagram with the emitted and received pulses, where: **304** represents the emitted pulse at the gas absorption peak wavelength; **305** represents four ideal replica pulses without any attenuation (one per sensor in the embodiment of **Figure 2****); 306** represents four replica pulses at the gas absorption peak wavelength with attenuation (one per sensor in the embodiment of **Figure 2****); 307** represents the emitted pulse at a different wavelength from the gas absorption peak wavelength; **308** represents four replica pulses outside the gas absorption peak wavelength with attenuation (one per sensor in the embodiment of **Figure 2****); 309** represents the same four replica pulses of **306;** and **310** represents the difference between two pulses from the same sensor, representing the true gas absorption at a gas monitoring point.

In an embodiment, the wavelength is periodically tuned at peak absorption, **302,** and outside, but close to the absorption peak, at a wavelength wherein the monitored gas suffers no absorption, **303,** as schematically identified in **Figure 3A****.** This is easy to accomplish by using the Laser driver, **202.** Referring to **Figure 3B****,** a first pulse, **304,** is emitted by the gas concentration interrogator, **201,** which is tuned to the absorption peak, **302.** Ideally, four replica pulses of identical amplitude, **305,** are sent back from the four sensors, **221.**However, the real pulse replicas, **306,** each coming from a respective sensor, has its amplitude reduced. To discard effects of attenuation of the fiber links, mirror differences and other issues from the attenuation of the pulses due to the absorption of the gas, one periodically sends a reference pulse, **307,** outside of the peak with no absorption, as identified in the schematic absorption curve, **301.** Thus, the amplitude difference between a replica pulse outside the absorption peak wavelength, and a replica pulse when laser is tuned to the absorption peak, for example at 1650.9 nm, gives rise to the actual value of absorption, **310.**

In an embodiment, the gas concentration is obtained by the Beer-Lambert law, as known by those skilled in the art. This makes the disclosed system self-referenced, and insensitive to other losses and issues not related to gas absorption itself. The alternating wavelength change does not need to be performed each time the replica signals tuned to the peak absorption wavelength, for instance, 1650.9 nm, return to the gas concentration interrogator, **201.** This can be done once every 5 measurements, once every 10 measurements or another appropriate frequency, as slow changes in the state of the optical network are expected.

**Figure 4** represents another embodiment of the gas concentration interrogator, where: **401** represents a first laser driver emitting pulses at the absorption peak wavelength; **402** represents a second laser driver emitting pulses outside the absorption peak wavelength; **403** represents a first DFB; **404** represents a second DFB; **405** represents a 1:99 optical splitter; **406** represents a reference absorption cell, such as a CH₄ reference absorption cell; **407** represents a first photodetector; **408** represents a second photodetector; **409** represents a 2x1 optical switch, and **410** represents an optical circulator.

**Figure 5** represents another embodiment of the gas concentration interrogator, where: **501** represents a first laser driver emitting pulses at the absorption peak wavelength; **502** represents a second laser driver emitting pulses outside the absorption peak wavelength; **503** represents a first DFB; **504** represents a second DFB; **505** represents a 1:99 optical splitter; **506** represents CH₄ reference absorption cell; **507** represents a first photodetector; **508** represents a first optical switch; **509** represents a second optical switch; **510** represents a 50:50 optical splitter; **511** represents an optical circulator; and **512** represents a second photodetector.

Two alternative embodiments, represented in **Figures 4** and **5****,** show two other forms to perform the change of wavelength, wherein both rely on a second laser source already tuned outside peak absorption. A possible embodiment, as shown in **Figure 4****,** uses a 2x1 optical switch, **409,** to define the path between the emitting laser driver, **401** or **402,** and the sensors. Another embodiment, shown in **Figure 5****,** uses two 1:1 optical switches, **508** and **509,** respectively, or equivalent devices and an optical splitter 50:50, **510,** to combine the signals which may come from laser driver **501** or laser driver **502,** depending on the state of the optical switch or switches.

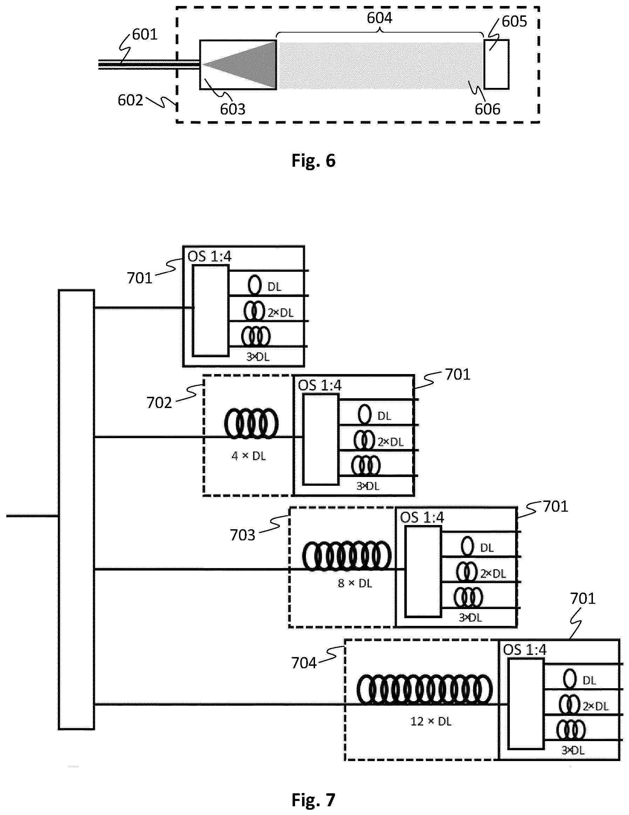

**Figure 6** represents an embodiment of the gas concentration sensor, where: **601** represents fiber optics cable; **602** represents the entirety of the gas concentration sensor, for instance for monitoring methane concentration; **603** represents a converging lens acting as an optical collimator and an optical focusing element; **604** represents a channel where the monitored gas flows through; **605** represents a mirror that reflects the emitted light from the optical collimator, also referred to as optical collimator, at a specific wavelength; and **606** represents a set of parallel rays of light passing through the channel wherein the monitored gas flows through.

In an embodiment, the sensor, schematically represented in **Figure 6****,** is usually an optical collimator, **603,** to expand the optical beam from the 9 µm diameter of the fiber core of the fiber, **601,** to a bigger cross section, in the order of millimeters. Note that the 9 mm diameter is the typical diameter of the core of a single-mode fiber. The difference in this sensor, is that mirror itself, **605,** is selective to reflect light around the wavelength corresponding to the peak of absorption; in the case of the methane gas, this peak at 1650.9 nm, covering the cases when the laser is tuned in the peak of the absorption line, and outside this peak when no absorption of the line occurs. That is, this mirror, **605,** reflects equally well these two wavelengths. However, this mirror, **605,** does not reflect the wavelengths where FBG interrogator and FBG sensors operate, usually in the range of 1510 nm to 1590 nm. In this case, the light, **606,** in these wavelengths is lost and just the reflection of the FBG sensor returns to FBG interrogator, as previously explained.

In another embodiment, the sensor may be a circular optical sensor, i.e. a sensor with a semi-circular converging lens and/or collimator, **603.** Herein, the optical beam is expanded by said optical collimator, **603.** Likewise, the sensor also comprises a channel where said monitored gas flows through, **604,** as well as a mirror, **605,** that reflects radiation of wavelength that is also absorbed by the gas being monitored, **302.**

In another embodiment, the sensor may be a micro-structured fiber optic without collimator. Herein, the sensor comprises a channel, **604,** and a mirror, **605,** but no optics element such as a collimator or converging lens is required.

There are other ways to build optical sensors with larger optical paths, and thus more sensitive to the gas, which can be used in the disclosed. Nonetheless, in the described embodiments, the mirror selection to be used in the optical sensor should consider the wavelengths that are and are not reflected.

**Figure 7** represents part of a different embodiment with a modular and scalable approach, where: **701** represents a module comprising an optical splitter of 1:4 and delay lines, similarly to the embodiments of **Figures 1** and **2****; 702** represents a delay line equivalent to four unitary delay lines; **703** represents a delay line equivalent to eight unitary delay lines; and **704** represents a delay line equivalent to twelve unitary delay lines.

In an embodiment, the combination of splitters of 1:4 and of DLs can be considered as distribution boxes, **701,** as per **Figure 7****,** but other combinations can be used. The exemplary embodiment shows how to achieve 16 divisions with increased optical delays varying from 0 to 15 DL steps, with steps of 1 DL. This is achieved by placing delay lines equivalent to four unitary delay lines, **702,** eight unitary delay lines, **703,** and twelve unitary delay lines, **704,** before each respective optical splitter, **701.** In this fashion, there is an economy of fiber optic necessary to achieve the delays, when compared with just one splitter of 1:16, in which each arm has a delay of 0 DL,1 DL, 2 DL, ...., 15 DL consuming an amount of fiber of 120 DL in total. With the embodiment represented in **Figure 7****,** the consumption of fiber is reduced to just 48 DL in total. Of course, in terms of cost, the user evaluates the total number of splitters and total of fiber use and their individual cost to decide how to implement the solution. The important aspect is that the proposed system enables this modularity and scalability. Observe that the extra DLs of 4xDL, **702,** 8xDL, **703,** and 12xDL, **704,** can be incorporated inside standardized distribution boxes or in the control room near the gas concentration interrogator. **Figure 7** exemplifies an embodiment to achieve a total of 16 ports, but other total number of ports can be used, such as 32, 64, 128, 256, and so on.

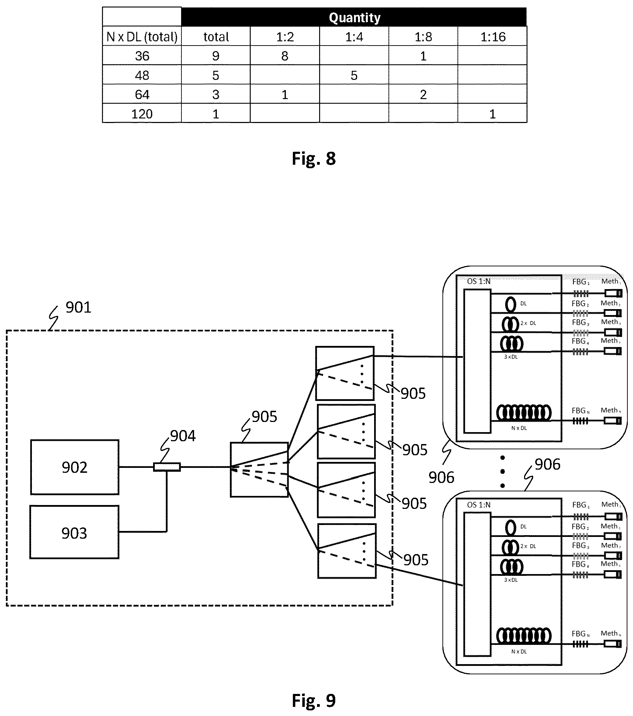

**Figure 8** represents possible combinations of number of splitters, its kinds and the respective total amount of delay line used in totals.

**Figure 9** represents an embodiment of the disclosed, wherein the signals emitted from the gas concentration and FBG interrogators are sent to various sub-networks of sensors with Optical Switches, where: **901** represents a control part of the optical circuit; **902** represents a gas concentration interrogator; **903** represents a FBG interrogator; **904** represents a WDM coupler; **905** represents an optical switch; and **906** represents a subnetwork of sensors and respective accompanying devices.

In an embodiment, as shown in **Figure 9****,** a combination of optical switches, **905,** can be used to achieve a large number of monitoring points. For example, using one optical switch of 1:4 and four optical switches of 1:128 and distribution boxes with, for example, 32 ports, one can reach the total number of 4 x 128 x 32 = 16384 monitoring points with the disclosed optical network, with both gas concentration sensors and FBG sensors, **906,** which is more than the 10000 point usually needed for monitoring an offshore platform. These optical switches are part of a control part of the optical circuit, and are preferably located in a control room. The number of optical switches, OS, DL and distribution boxes is completely scalable and modular and a decision that the user can adequate to their needs.

In an embodiment, the laser is a modulation-free laser for continuous wave operations.

The disclosed allows the lowering of the required sampling rate and/or allows reduction of the delay line, which means fewer optical fibers and a lower cost. Furthermore, smaller delay lines take up less physical space, making it easier to control sizes in production. For example, cutting an optical cable with many fibers (>48) producing 48 exactly identical pieces of fiber: elimination of overlapping return pulses. In other words (allows for shorter fiber lengths, which can be realized with accurate, equal lengths). This also simplifies electronics, and permits the elimination of a WDM per sensor due to the use of selective mirror (reflection only in the absorption band, e.g., 1650 nm).

This disclosure is co-financed by Component 5 - Capitalization and Business Innovation of core funding for Technology and Innovation Centres (CTI), integrated in the Resilience Dimension of the Recovery and Resilience Plan within the scope of the Recovery and Resilience Mechanism (MRR) of the European Union (EU), framed in the Next Generation EU, for the period 2021 - 2026, with reference 21.

This disclosure is also co-financed by Component 5 - Capitalization and Business Innovation, integrated in the Resilience Dimension of the Recovery and Resilience Plan within the scope of the Recovery and Resilience Mechanism (MRR) of the European Union (EU), framed in the Next Generation EU, for the period 2021 - 2026, within project ATE, with reference 56. Claudio Floridia and Luís Coelho are financed by FCT - Portuguese Foundation for Science and Technology, under grants: doi.org/10.54499/CEECINST/00033/2021/CP2825/CT0001 and CEECIND/00471/2017.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable. The following dependent claims further set out particular embodiments of the disclosure.

## Claims

1. System for passive optical monitoring of a gaseous substance at a plurality of measuring points, comprising:
a laser interrogator (101, 201, 902) for emitting (103) a laser pulse and receiving (104) laser pulses;
a plurality of fibre-optic lines (100, 109, 110, 111, 200, 217, 218, 219), each fibre-optic line for a measuring point;
an optical splitter (108, 216) connected to the laser interrogator for splitting the emitted laser pulse into the plurality of fibre-optic lines;
a plurality of gas concentration sensors, each connected to a corresponding fibre-optic line; and
a data processor configured for determining a presence at a measuring point of the gaseous substance to be monitored from an attenuation of a corresponding received laser pulse;
wherein each fibre-optic line has a unique delay in respect to the other fibre-optic lines;
wherein each gas concentration sensor comprises:
a channel for receiving the gaseous substance to be monitored by absorption by the gaseous substance of a corresponding split laser pulse;
an end of a corresponding fibre-optic line for sending the split laser pulse through the channel; and
a mirror for reflecting the emitted laser pulse back into the corresponding fibre-optic line.

2. System according to the previous claim, comprising:
a Fiber Bragg Grating, FBG, interrogator (102, 213, 903), for emitting (105, 214) and receiving (106, 215) an FBG signal,
a plurality of FBG sensors (112, 220) arranged for measuring a physical parameter by receiving the emitted FBG signal and reflecting the emitted FBG signal, according to the physical parameter to be measured, towards the FBG interrogator (102, 213, 903), and
a Wavelength Division Multiplexing, WDM, coupler (107, 212, 904) arranged for multiplexing the emitted FBG signal and the emitted laser pulse into the optical splitter, and for demultiplexing the reflected FBG signals and the reflected laser pulses from the optical splitter;
wherein the emitted FBG signal and the emitted laser pulse have different wavelengths;
wherein the mirror of the gas concentration sensor is configured to not reflect the emitted FBG signal; and
wherein the physical parameter measured by the plurality of FBG sensors (112, 220) is selected from: temperature, strain, pressure, water level, current, a second gas substance concentration, or combinations thereof, in particular, the second gas substance being Hydrogen.

3. System according to any of the previous claims, wherein the laser interrogator (101, 201, 902) is configured to emit and receive a calibration laser pulse (303) having a wavelength that does not correspond to an absorption band of the gaseous substance, and the data processor is further configured for calibrating the presence at the measuring point of the gaseous substance to be monitored from an attenuation of the received calibration laser pulse.

4. System according to any of the previous claims, wherein one of said fibre-optic lines has no added delay line and each of the remaining fibre-optic lines comprises a delay line wherein each delay line has a unique delay in respect to the other delay lines.

5. System according to any of the previous claims, wherein the laser interrogator (101, 201, 902) comprises:
one or two Distributed Feedback Lasers, DFL (203, 403, 404, 503, 504), for generating a laser signal;
one or two laser drivers (202, 401, 402, 501, 502) for controlling each of the one or DFL (203, 403, 404, 503, 504) by sending an electrical control signal;
a gaseous-substance reference absorption cell (205, 406, 506), comprising the gaseous substance to be monitored by absorption of the gaseous substance of a corresponding generated laser signal;
a first photodetector (206, 407, 507) arranged for detecting the generated laser signal from the gaseous-substance reference absorption cell (205, 406, 506), and for converting said portion of laser signal into an electrical signal;
a second photodetector (211, 408, 512) arranged for detecting at least one reflected laser pulse (104) and converting said received laser pulse into at least one electrical pulse signal;
a signal acquisition processor (207) configured to acquire the electric signal from the first photodetector (206, 407, 507), and to acquire the at least one electrical signal pulse from the second photodetector (211, 408, 512);
the data processor configured to quantify the concentration of the monitored gas from the acquired electric signal from the second photodetector, and to calibrate the one or two laser drivers (202, 401, 402, 501, 502) from the acquired electric signal from the first photodetector;
a pulse generator (209) for generating a further electrical signal pulse of a predefined pulse width, and for triggering the signal acquisition processor (207);
a fast switch (208, 409, 508, 509) for generating the laser pulse from the laser signal generated by the one or two DFL (203, 403, 404, 503, 504), wherein the laser pulse has the same pulse width as the further electrical signal;
a first optical splitter (204, 405, 505) for splitting the generated laser signal from the one or two DFL (203, 403, 404, 503, 504) into two identical outputting laser signals, wherein one of the two identical outputting laser signals is sent to the fast switch, and wherein another of the two identical outputting laser signals is sent to the gaseous-substance reference absorption cell (205, 406, 506); and
an optical circulator (210, 410, 511) arranged for receiving the generated laser pulse (103) from the fast switch (208, 409, 508, 509) and transmitting said generated laser pulse (103) to the WDM coupler (107, 212, 904), and for receiving the demultiplexed laser pulses (104) and transmitting said demultiplexed laser pulses (104) to the second photodetector (211, 408, 512).

6. System according to any of the previous claims, comprising a module (701) further comprising one or more optical splitters (108, 216) for connecting the WDM coupler (107, 212, 904) to the two or more gas concentration sensor (113, 221, 602) and the two or more FBG sensors (112, 220), wherein the splitter has a number of outputs identical to the number of the two or more gas concentration sensor (113, 221, 602) and the two or more FBG sensors (112, 220), and in particular wherein said module (701) further comprises at least one delay line (109, 110, 111, 217, 218, 219) connected between the one or more optical splitters (108, 216) and each of the two or more FBG sensors (112, 220), for delaying the receiving laser pulses (104) and the receiving light pulses (106, 215).

7. System according to any of the previous claims, wherein each gas concentration sensor of plurality of gas concentration sensors further comprises an optic, arranged on an end of the corresponding fibre-optic line, for sending the split laser pulse through the channel to be reflected by the mirror back into the corresponding fibre-optic line.

8. System according to any of the previous claims, wherein the mirror (605) of the at least one gas concentration sensor (113, 221, 602) reflects rays of the at least two distinct wavelengths, emitted by the one or two DFL (203, 403, 404, 503, 504).

9. System according to any of the previous claims, wherein the first optical splitter (204, 405, 505) for of the gas concentration interrogator (101, 201, 902) divides the laser signal, generated by one of the one or two DFL (203, 403, 404, 503, 504), into two outputting laser signals, wherein:
one of the two outputting laser signals, comprises a smaller power percentage of the laser signal generated by the one of the one or two DFL (203, 403, 404, 503, 504), which is sent to the gas reference absorption cell (205, 406, 506), and
another of the two outputting laser signals, comprises a larger power percentage of the laser signal generated by the one of the one or two DFL (203, 403, 404, 503, 504), which is sent to the fast switch (208, 409, 508, 509).

10. System according to claims 4 and 5, further comprising two or more optical splitters (701), and further delay lines (702, 703, 704) arranged between the WDM coupler (107, 212, 904) and the module (701), for delaying the receiving laser pulses (104) and the receiving light pulses (106, 215).

11. System according to any of the previous claims, wherein the gas concentration interrogator (101, 201, 902) comprises two DFL (403, 404, 503, 504) and two laser drivers (401, 402, 501, 502), wherein:
the laser signal generated by the one of the two DFL (403, 503) has an identical wavelength of an absorption peak (302) of the gas; and
the laser signal of the another of the two DFL (404, 504) has a different wavelength of the absorption peak (303) of the gas; and
each of the two laser drivers (401, 402, 501, 502) controls each of the two DFL (403, 404, 503, 504).

12. System according to the previous claim, wherein the gas concentration interrogator (101, 201, 902) further comprises two fast switches (508, 509) and a second optical splitter (510), wherein:
one of the two fast switches (508) is connected to the first optical splitter (204, 405, 505) of the gas concentration interrogator (101, 201, 902), for generating a first laser pulse from the laser signal generated by the one of the two DFL (403, 503);
another of the two fast switches (509) is connected to another of the two DFL (404, 504) of the gas concentration interrogator (101, 201, 902), for generating a second laser pulse from the laser signal generated by the another of the two DFL (404, 504); and
a second optical splitter (510) connecting each of the two fast switches (508, 509) to the optical circulator (210, 410, 511).

13. System according to claim 10, wherein the fast switch (409) of the gas concentration interrogator (101, 201, 902) receives the laser signal, with a wavelength identical to that of the absorption peak (302) of the gas, from the first optical splitter (204, 405, 505), generated by the one of the two DFL (403, 503), and receives the laser signal, with a different wavelength to that of the absorption peak (303) of the gas, from the other of the two DFL (404, 504).

14. Method for passive optical monitoring of a gaseous substance at a plurality of measuring points, comprising the following steps:
generating a laser pulse in the gas concentration interrogator (101, 201, 902) with an identical wavelength of an absorption peak (302) of the gas;
converting the at least one demultiplexed laser pulse with an identical wavelength of an absorption peak (302) of the gas, from the at least one gas concentration sensor (113, 221, 602) into at least one electrical pulse signal with the second photodetector (211, 408, 512);
sending the at least one electrical pulse signal to the signal acquisition processor (207);
generating a laser pulse in the gas concentration interrogator (101, 201, 902) different wavelength of the absorption peak (303) of the gas;
converting the at least one receiving laser pulse with another wavelength (303), from the at least one gas concentration sensor (113, 221, 602) into at least one electrical signal with the second photodetector (211, 408, 512).

15. The method according to the previous claim, wherein the generation of a laser pulse (103) for the measurement of a gas concentration comprises the following steps:
sending an electrical control signal from the one or two laser drivers (202, 401, 402, 501, 502) to the one or two DFL (203, 403, 404, 503, 504) for generating a laser signal;
sending the generated laser signal from the one or two DFL (203, 403, 404, 503, 504) to the first optical splitter (204, 405, 505), for dividing the generated laser signal into two outputting laser signals, wherein:
one of the two outputting laser signals comprises a smaller power percentage relative the laser signal generated, and
another of the two outputting laser signals comprises a larger power percentage relative the laser signal, which is sent to the fast switch (208, 409, 508, 509); and
generating a laser pulse from the laser signal generated by the one or two DFL (203, 403, 404, 503, 504), wherein the laser pulse has the same pulse width as the further electrical signal pulse generated from the pulse generator (209).
